(19) Europäisches Patentamt
European Patent Office
Office européen des brevets

(11) Veröffentlichungsnummer: **0 158 957**
**B1**

# (12) EUROPÄISCHE PATENTSCHRIFT

(45) Veröffentlichungstag der Patentschrift: **18.01.89**

(51) Int. Cl.[4]: **C 07 D 295/20**, C 07 C 127/22, C 07 C 127/24, C 07 C 155/02

(21) Anmeldenummer: **85104258.0**

(22) Anmeldetag: **09.04.85**

(54) **Allophanat-Derivate.**

(30) Priorität: **19.04.84 DE 3414881**

(43) Veröffentlichungstag der Anmeldung: **23.10.85 Patentblatt 85/43**

(45) Bekanntmachung des Hinweises auf die Patenterteilung: **18.01.89 Patentblatt 89/3**

(84) Benannte Vertragsstaaten: **CH DE FR GB IT LI**

(56) Entgegenhaltungen: **DE-A- 2 447 626**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Führer, Wolfgang, Dr., Wehrstrasse 28, D-5202 Hennef 1 (DE)**
Erfinder: **Kühle, Engelbert, Dr., von-Bodelschwingh-Strasse 42, D-5060 Bergisch-Gladbach 2 (DE)**
Erfinder: **Hänssler, Gerd, Dr., Heymannstrasse 40, D-5090 Leverkusen 1 (DE)**

**EP 0 158 957 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Allophanat-Derivate, Verfahren zu ihrer Herstellung und ihre Verwendung als Schädlingsbekämpfungsmittel.

Es ist bereits bekannt, dass verschiedene Kohlensäurederivate oder deren Metallkomplexe, z.B. das Zinkethylen-1,2-bis-dithiocarbamat, sich zum Schutz von Kulturpflanzen vor dem Befall durch Schadpilze eignen (vgl. z.B. R. Wegler, «Chemie der Pflanzenschutz- und Schädlingsbekämpfungsmittel», Bd. 2, Seite 65, Springer Verlag Berlin (1970)).

Die Wirkung dieser Verbindungen kann jedoch unter bestimmten Bedingungen, z.B. bei niedrigen Aufwandmengen und -konzentrationen, nicht immer zufriedenstellend sein.

Weiterhin ist der N-Carbethoxy-N-methyl-N'-phenoxycarbonylharnstoff als Ausgangsprodukt zur Herstellung von Penicillinen bekannt (vgl. DE-A-2 447 626).

Es wurden neue Allophanat-Derivate der Formel (I)

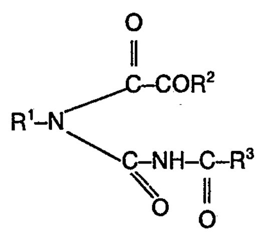

(I)

in welcher

$R^1$ und $R^2$ gleich oder verschieden sind und für gegebenenfalls durch $C_{1-4}$-Alkoxy, $C_{1-4}$-Alkylthio und/oder Halogen substituiertes $C_1$–$C_{10}$-Alkyl oder $C_2$–$C_{10}$-Alkenyl oder $C_2$–$C_{10}$-Alkinyl stehen, ferner für gegebenenfalls durch $C_1$–$C_6$-Alkyl substituiertes $C_5$–$C_{10}$-Cycloalkyl, für gegebenenfalls im Arylteil durch Halogen, Nitro, $C_1$–$C_6$-Alkyl, Cyano und/oder Trifluormethyl substituiertes Aralkyl mit $C_6$–$C_{10}$ im Arylteil und $C_1$–$C_4$ im Alkylteil stehen, für gegebenenfalls durch Halogen, Nitro, Cyano, $C_1$–$C_6$-Alkyl, $C_1$–$C_6$-Alkoxy, und/oder Trifluormethyl substituiertes $C_6$–$C_{10}$-Aryl stehen,

$R^3$ für die Reste $-XR^4$ oder $-NR^5R^6$ steht, wobei

$R^4$ für $C_{1-10}$-Alkyl, für $C_{1-4}$-Alkoxy-$C_{1-4}$-alkyl oder für gegebenenfalls ein- bis fünffach, gleich oder verschieden durch Halogen, $C_{1-4}$-Alkyl oder Halogenalkyl mit 1 oder 2 Kohlenstoffatomen und 1 bis 5 gleichen oder verschiedenen Halogenatomen substituiertes Aryl mit 6 bis 10 Kohlenstoffatomen steht,

X für Sauerstoff oder Schwefel steht und

$R^5$ und $R^6$ gleich oder verschieden sind und für Wasserstoff, für $C_{1-10}$-Alkyl oder für gegebenenfalls ein- bis fünffach, gleich oder verschieden durch Halogen, $C_{1-4}$-Alkyl, Halogenalkyl oder Halogenalkoxy mit je 1 oder 2 Kohlenstoffatomen und 1 bis 5 gleichen oder verschiedenen Halogenatomen substituiertes Aryl mit 6 bis 10 Kohlenstoffatomen stehen oder

$R^5$ und $R^6$ gemeinsam mit dem Stickstoffatom, an dem sie stehen, für einen 5- bis 7-gliedrigen gesättigten, gegebenenfalls durch $C_{1-4}$-Alkyl substituierten Heterocyclus stehen, der durch ein weiteres Heteroatom unterbrochen sein kann, ausgenommen die Verbindung N-Carbethoxy-N-methyl-N'-phenoxycarbonylharnstoff, gefunden.

Man erhält die neuen Allophanat-Derivate der Formel (I)

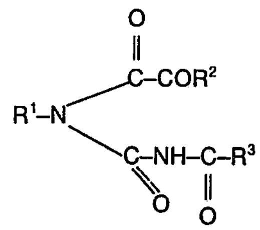

(I)

in welcher

$R^1$ und $R^2$ gleich oder verschieden sind und für gegebenenfalls durch $C_{1-4}$-Alkoxy, $C_{1-4}$-Alkylthio und/oder Halogen substituiertes $C_1$–$C_{10}$-Alkyl oder $C_2$–$C_{10}$-Alkenyl oder $C_2$–$C_{10}$-Alkinyl stehen, ferner für gegebenenfalls durch $C_1$–$C_6$-Alkyl substituiertes $C_5$–$C_{10}$-Cycloalkyl, für gegebenenfalls im Arylteil durch Halogen, Nitro, $C_1$–$C_6$-Alkyl, Cyano und/oder Trifluormethyl substituiertes Aralkyl mit $C_6$–$C_{10}$ im Arylteil und $C_1$–$C_4$ im Alkylteil stehen, für gegebenenfalls durch Halogen, Nitro, Cyano, $C_1$–$C_6$-Alkyl, $C_1$–$C_6$-Alkoxy, und/oder Trifluormethyl substituiertes $C_6$–$C_{10}$-Aryl stehen,

$R^3$ für die Reste $-XR^4$ oder $-NR^5R^6$ steht, wobei

$R^4$ für $C_{1-10}$-Alkyl, für $C_{1-4}$-Alkoxy-$C_{1-4}$-alkyl oder für gegebenenfalls ein- bis fünffach, gleich oder verschieden durch Halogen, $C_{1-4}$-Alkyl oder Halogenalkyl mit 1 oder 2 Kohlenstoffatomen und 1 bis 5 gleichen oder verschiedenen Halogenatomen substituiertes Aryl mit 6 bis 10 Kohlenstoffatomen steht,

X für Sauerstoff oder Schwefel steht und

$R^5$ und $R^6$ gleich oder verschieden sind und für Wasserstoff, für $C_{1-10}$-Alkyl oder für gegebenenfalls ein- bis fünffach, gleich oder verschieden durch Halogen, $C_{1-4}$-Alkyl, Halogenalkyl oder Halogenalkoxy mit je 1 oder 2 Kohlenstoffatomen und 1 bis 5 gleichen oder verschiedenen Halogenatomen substituiertes Aryl mit 6 bis 10 Kohlenstoffatomen stehen oder

$R^5$ und $R^6$ gemeinsam mit dem Stickstoffatom, an dem sie stehen, für einen 5- bis 7-gliedrigen gesättigten, gegebenenfalls durch $C_{1-4}$-Alkyl substituierten Heterocyclus stehen, der durch ein weiteres Heteroatom unterbrochen sein kann, ausgenommen die Verbindung N-Carbethoxy-N-methyl-N'-phenoxycarbonylharnstoff,

wenn man

a) N-Isocyanatocarbonyl-carbamate der Formel (II)

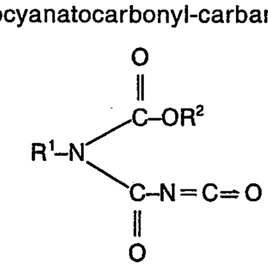

(II)

in welcher

$R^1$ und $R^2$ die oben angegebene Bedeutung haben, mit Verbindungen der Formel (III)

$$MR^3 \qquad (III)$$

in welcher

$R^3$ die oben angegebene Bedeutung hat und

M für Wasserstoff oder ein Metallkationäquivalent, vorzugsweise ein Alkalimetalläquivalent, wie Natrium und Kalium, steht,

gegebenenfalls in Gegenwart eines basischen Hilfsstoffes und gegebenenfalls in Gegenwart eines Verdünnungs- oder Lösungsmittels umsetzt oder wenn man

b) N-Halogencarbonyl-urethane der Formel (IV)

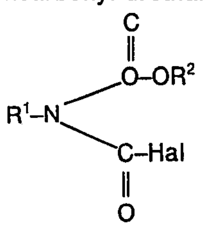

(IV)

in welcher

$R^1$ und $R^2$ die oben angegebene Bedeutung haben und

Hal für Halogen, vorzugsweise Chlor, steht mit Verbindungen der Formel (VI)

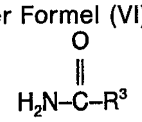

(V)

in welcher

$R^3$ die oben angegebene Bedeutung hat,

gegebenenfalls in Gegenwart eines Lösungs- oder Verdünnungsmittels und gegebenenfalls in Gegenwart einer Base umsetzt.

Die neuen Allophanat-Derivate der Formel (I) weisen vor allem fungizide Eigenschaften auf. Dabei zeigen die meisten der erfindungsgemässen Verbindungen eine höhere fungizide Wirksamkeit als die aus dem Stand der Technik vorbekannte fungizid wirksame Verbindung, wie z.B. das Zinkethylen-bis-dithiocarbamat. Die erfindungsgemässen Verbindungen stellen somit eine Bereicherung der Technik dar.

Die erfindungsgemässen Allophanat-Derivate sind durch die Formel (I) allgemein definiert.

Bevorzugt sind Verbindungen der Formel (I), bei denen

$R^1$ und $R^2$ gleich oder verschieden sind und für gegebenenfalls durch Methoxy, Ethoxy, n- und i-Propoxy, Methylthio, Ethylthio, n- und i-Propylthio, Fluor und/oder Chlor substituiertes $C_1$–$C_6$-Alkyl oder $C_3$–$C_5$-Alkenyl oder $C_3$–$C_5$-Alkinyl stehen, für gegebenenfalls durch Methyl, Ethyl, n- und i-Propyl, n-, s-, i- und t-Butyl substituiertes $C_5$- oder $C_6$-Cycloalkyl, für gegebenenfalls im Arylteil durch Fluor, Chlor, Nitro, Cyano, Methyl, Ethyl, n- und i-Propyl, n-, s-, i- und t-Butyl und Trifluormethyl substituiertes Arylalkyl, vorzugsweise Benzyl und Phenylethyl stehen, für gegebenenfalls durch Fluor, Chlor, Nitro, Methyl, Ethyl, n- und i-Propyl, n-, i-, s- und t-Butyl, Methoxy, Ethoxy, n- und i-Propoxy, und/oder Trifluormethyl substituiertes $C_6$–$C_{10}$-Aryl, insbesondere Phenyl und Naphthyl stehen,

$R^3$ für die Reste $-XR^4$ oder $-NR^5R^6$ steht, wobei

$R^4$ für $C_{1-10}$-Alkyl, für $C_{1-3}$-Alkoxy-$C_{1-4}$-alkyl oder gegebenenfalls ein- bis dreifach, gleich oder verschieden durch Chlor, Methyl, Ethyl oder Trihalogenmethyl substituiertes Phenyl steht,

X für Sauerstoff oder Schwefel steht,

$R^5$ und $R^6$ gleich oder verschieden sind und für Wasserstoff, $C_{1-6}$-Alkyl oder für gegebenenfalls ein- bis dreifach, gleich oder verschieden durch Chlor, Methyl, Ethyl, Trihalogenmethyl, Trihalogenmethoxy substituiertes Phenyl stehen oder

$R^5$ und $R^6$ gemeinsam mit dem Stickstoffatom, an dem sie stehen, einen gesättigten, gegebenenfalls ein- oder zweifach durch Methyl substituierten 5- bis 7-gliedrigen Heterocyclus bilden, der durch ein Sauerstoffatom unterbrochen sein kann, ausgenommen die Verbindung N-Carbethoxy-N-methyl-N'-phenoxycarbonylharnstoff.

Ganz besonders bevorzugt sind Verbindungen der Formel (I), in denen

$R^1$ für $C_{1-6}$-Alkyl, Cyclopentyl, Cyclohexyl, gegebenenfalls ein- oder zweifach durch Chlor, Methyl, tert.-Butyl oder Trifluormethyl substituiertes Phenyl oder Benzyl steht,

$R^2$ für $C_{1-4}$-Alkyl, für gegebenenfalls ein- bis dreifach, gleich oder verschieden durch Methyl, Chlor, und iso-Propoxy substituiertes Phenyl steht,

$R^3$ für die Reste $-XR^4$ oder $-NR^5R^6$ steht, wobei

$R^4$ für $C_{1-10}$-Alkyl, für $C_{1-3}$-Alkoxy-$C_{1-4}$-alkyl oder gegebenenfalls ein- bis dreifach, gleich oder verschieden durch Chlor, Methyl, Ethyl oder Trifluormethyl substituiertes Phenyl steht,

X für Sauerstoff oder Schwefel steht,

$R^5$ und $R^6$ gleich oder verschieden sind und für Wasserstoff, $C_{1-6}$-Alkyl oder für gegebenenfalls ein- bis dreifach, gleich oder verschieden durch Chlor, Methyl, Ethyl, Trifluormethyl oder Trifluormethoxy substituiertes Phenyl stehen oder

$R^5$ und $R^6$ gemeinsam mit dem Stickstoffatom, an dem sie stehen, für einen gegebenenfalls ein- oder zweifach durch Methyl substituierten Morpholinrest, Pyrrolidinrest, Hexamethylenimidrest oder Piperidinrest stehen, ausgenommen die Verbindung N-Carbethoxy-N-methyl-N'-phenoxycarbonylharnstoff.

Verwendet man bei der Herstellung der erfindungsgemässen Verbindungen der Formel (I) nach der Verfahrensvariante a) N-Isocyanatocarbonyl-N-3-methylphenyl-carbamidsäure-4-kresylester und Pyrrolidin und nach der Verfahrensvariante b) N-Chlorcarbonyl-N-phenyl-ethylurethan und N,N-Di-methyl-harnstoff als Ausgangskomponenten, so können die Reaktionsabläufe durch die folgenden Formelschemata wiedergegeben werden:

a)

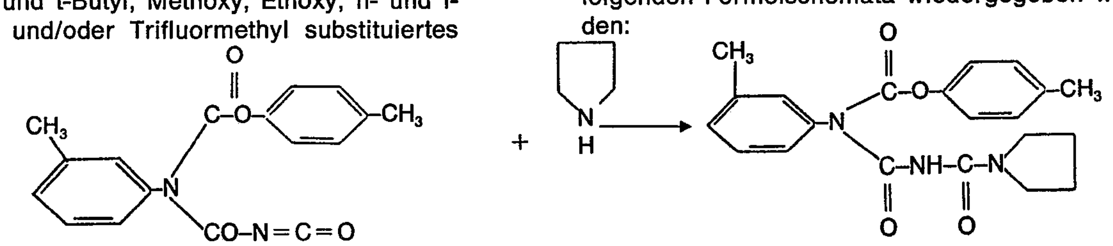

b) 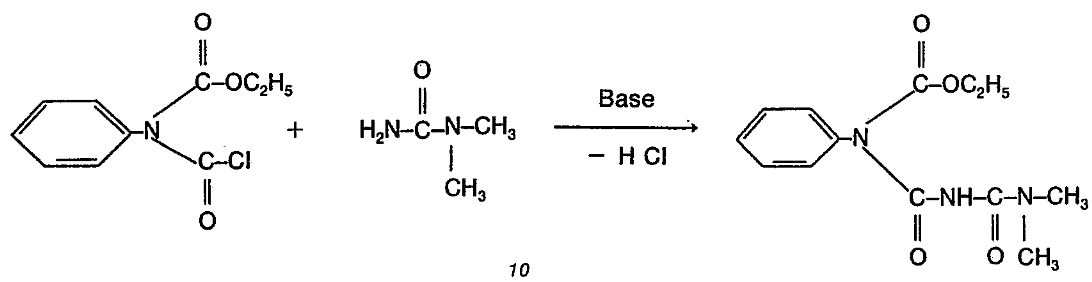

Die als Ausgangsstoffe für die Herstellung der erfindungsgemässen Verbindungen der Formel (I) benötigten N-Isocyanatocarbonylcarbamate sind durch die Formel (II) allgemein definiert. In dieser Formel (II) haben $R^1$ und $R^2$ die vorher unter der Formel (I) genannten Bedeutungen. Diese Verbindungen sind aus der DE-OS 3 146 230 bekannt, ebenso wie ihre Herstellung z.B. aus geeigneten N-substituierten Carbaminsäureestern mit Chlorcarbonylisocyanat bei Temperaturen zwischen 50 und 200 °C in einem Lösungsmittel. Die ebenfalls für die Variante a) benötigten Ausgangsstoffe der Formel (III), in der $R^3$ und M die bei der Formel (I) gegebenen Bedeutungen haben, sind allgemein bekannte und käuflich zu erwerbende Grundchemikalien wie Alkohole, Thiole oder Amine.

Die in der Verfahrensvariante b) als Ausgangsverbindungen einzusetzenden N-Halogencarbonyl-urethane sind durch die Formel (IV) allgemein definiert, wobei $R^1$ und $R^2$ die bei der Formel (I) gegebenen Bedeutungen haben und Hal für Halogen, vorzugsweise Chlor, steht. Diese Verbindungen sind grösstenteils aus der DE-OS 2 257 344 bekannt und können z.B. aus Iminokohlensäureestern mit Phosgen in Lösungsmitteln, wie z.B. Cyclohexan, Toluol oder Chlorbenzol, bei Temperaturen zwischen 20 und 120 °C erhalten werden. Die dazu benötigten Iminokohlensäureester sind ebenfalls bekannt (vgl. z.B. E. Kühle et al., «Angewandte Chemie» 81 (1969) 18ff).

Die bei der Verfahrensvariante b) ausserdem als Ausgangsverbindungen benötigten Stoffe werden durch die Formel (V) allgemein definiert. Darin hat $R^3$ die bei Formel (I) angegebene Bedeutung. Diese Harnstoffe, Carbamin- und Thiocarbaminsäureester sind allgemein bekannte Verbindungen. Die erfindungsgemässen Verfahrensvarianten a) und b) werden vorzugsweise in Anwesenheit eines Lösungs- oder Verdünnungsmittels durchgeführt. Hierbei kommen praktisch alle inerten, aprotischen organischen Solventien in Frage. Vorzugsweise verwendbar sind aliphatische und aromatische, gegebenenfalls halogenierte Kohlenwasserstoffe, wie Pentan, Hexan, Heptan, Cyclohexan, Benzol, Toluol, Xylol, Methylenchlorid, Chloroform, Tetrachlorkohlenstoff, Chlorbenzol und Dichlorbenzol, Ether, wie Diethylether, Diisopropylether, 1,2-Dimethoxy-ethan, Tetrahydrofuran und Dioxan, Ester, wie Essigsäuremethylester und -ethylester, Nitrile, wie Acetonitril und Propionitril, Amide, wie Dimethylformamid und Dimethylacetamid, sowie Dimethylsulfoxid und Sulfolan.

Die Umsetzung nach dem erfindungsgemässen Verfahren a) kann sowohl in Anwesenheit als auch in Abwesenheit von Säurebindemitteln vorgenommen werden. Arbeitet man in Gegenwart von Säurebindemitteln, so kommen als derartige Säureakzeptoren praktisch alle üblichen Säurebindemittel in Betracht.

Besonders bevorzugt verwendet sind Alkalimetall- und Erdalkalimetallhydroxide, wie z.B. Natrium-, Kalium- und Calcium-hydroxid, Alkalimetall- und Erdalkalimetallcarbonate bzw. -hydrogencarbonate, wie z.B. Natriumcarbonat und -hydrogencarbonat, Kaliumcarbonat und Calciumcarbonat, sowie aliphatische, aromatische und heterocyclische Amine, wie z.B. Trimethyl-, Triethyl-, Tripropyl- und Tributyl-amin, N,N-Dimethyl-benzylamin, Pyridin, 2-Methylpyridin, 2,4,6-Trimethylpyridin, 2-Methyl-5-ethyl-pyridin, Diazabicyclooctan (DABCO), Diazabicyclononen (DBN) und Diazabicycloundecen (DBU).

Bei der Verfahrensvariante b) wird bevorzugt in Gegenwart eines der oben genannten Säurebindemittels gearbeitet.

Die Reaktionstemperaturen können bei den Verfahren a) und b) innerhalb eines grösseren Bereiches variiert werden. Im allgemeinen arbeitet man bei Temperaturen zwischen −50 °C und +200 °C, vorzugsweise zwischen −20 °C und +150 °C.

Die Verfahren a) und b) werden im allgemeinen bei Normaldruck durchgeführt.

Zur Durchführung des erfindungsgemässen Verfahrens a) setzt man pro Mol an Ausgangsverbindung der Formel (II) im allgemeinen zwischen 0,8 und 4,0 Mol, vorzugsweise zwischen 0,9 und 3,0 Mol Ausgangsverbindung der Formel (III) ein. Arbeitet man in Gegenwart von Säurebindemitteln, so werden diese in äquivalenter Menge oder auch in einem Überschuss, bezogen auf Verbindungen der Formel (II), eingesetzt. Die Durchführung der Umsetzung erfolgt beim Arbeiten ohne Säurebindemittel im allgemeinen in der Weise, dass man die Komponenten zusammengibt und reagieren lässt. Arbeitet man in Gegenwart eines Säurebindemittels, so werden die Komponenten gegebenenfalls unter Kühlung zusammengegeben, und das Reaktionsgemisch wird danach gegebenenfalls bei erhöhter Temperatur gerührt. Die anschliessende Aufarbeitung erfolgt nach üblichen Methoden. Im allgemeinen geht man so vor, dass man den festen Rückstand abfiltriert, mit einem organischen Lösungsmittel extrahiert und die vereinigten organischen Phasen wäscht und einengt. Es ist jedoch auch möglich, das Reaktionsgemisch nach beendeter Umsetzung mit Wasser zu verdünnen, das entstehende Gemisch mit einem mit Wasser wenig mischbaren organischen Lösungsmittel zu extrahieren, die vereinigten organischen Phasen zu waschen und dann einzuengen. Die dabei anfallenden Produkte können durch übliche Massnahmen, wie Umkristallisation oder auf chromatographischem Wege, von eventuell noch enthaltenen Verunreinigungen befreit werden.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Botrytis-Arten, wie beispielsweise Botrytis cinerea;

Plasmopara-Arten, wie beispielsweise Plasmopara viticola;

Uromyces-Arten, wie beispielsweise Uromyces appendiculatus;

Sphaerotheca-Arten, wie beispielsweise Sphaerotheca fuliginea;

Venturia-Arten, wie beispielsweise Venturia inaequalis;

Podosphaera-Arten, wie beispielsweise Podosphaera leucotricha;

Phytophthora-Arten, wie beispielsweise Phytophthora infestans;

Erysiphe-Arten, wie beispielsweise Erysiphe graminis;

Puccinia-Arten, wie beispielsweise Pucciria recondita;

Fusarium-Arten, wie beispielsweise Fusarium culmorum;

Ustilago-Arten, wie beispielsweise Ustilago nuda oder avenae;

Septoria-Arten, wie beispielsweise Septoria nodorum;

Tilletia-Arten, wie beispielsweise Tilletia caries;

Xanthomonas-Arten, wie beispielsweise Xanthomonas oryzae;

Pseudomonas-Arten, wie beispielsweise Xanthomonas lachrymans;

Pyricularia-Arten, wie beispielsweise Pyricularia oryzae;

Pellicularia-Arten, wie beispielsweise Pellicularia sasakii;

Pyrenophora-Arten, wie beispielsweise Pyrenophora teres (Konidienform: Drechslera, Syn: Helminthosporium);

Leptosphaeria-Arten, wie beispielsweise Leptosphaeria nodorum;

Cochliobolus-Arten, wie beispielsweise Cochliobolus sativus

(Konidienform: Drechslera, Syn: Helminthosporium)

und

Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweisshydrolysate. Als

Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Giessen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Einige der erfindungsgemässen Verbindungen zeigen auch eine selektiv-herbizide Wirksamkeit oder wurzelsystemisch insektizide Wirksamkeit.

Herstellungsbeispiele

Beispiel 1:

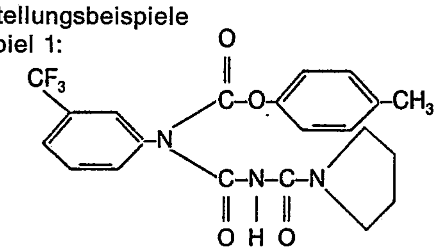

16 g (0,044 Mol) N-Isocyanatocarbonyl-N-3-trifluormethylphenylcarbamidsäure-4-kresylester werden in 100 ml Dichlormethan gelöst und tropfenweise mit einer Lösung von 31 g (0,044 Mol) Pyrrolidin in 50 ml Dichlormethan versetzt. Nach Abklingen der exothermen Reaktion lässt man über Nacht bei Raumtemperatur stehen, filtriert dann von der Trübung ab und dampft das Filtrat im Vakuum ein. Es verbleiben 19,1 g an $N^2$-Pyrrolidinocarbonyl-$N^1$-3-trifluormethyl-phenyl-allophansäure-4-kresylester in Form eines farblosen viskosen Öls (quantitative Ausbeute) mit einem Brechungsindex $n_D^{20}$ = 1.5080.

Herstellung des Vorproduktes:

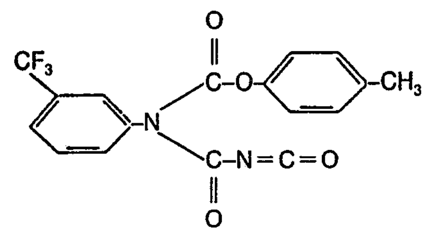

Verfahren nach DE-OS 3 146 230

145 g (0,49 Mol) 3-Trifluormethylphenylcarbamidsäure-4-kresylester, erhalten aus 3-Trifluormethylphenylisocyanat und p-Kresol (Fp. 125–127 °C), werden in 600 ml Chlorbenzol suspendiert und mit 58 g (0,55 Mol) Chlorcarbonylisocyanat unter Feuchtigkeitsausschluss 7 Stunden zum Sieden erhitzt. Danach wird das Lösungsmittel im Vakuum abdestilliert, wobei 171 g (96% der Theorie) eines gelblich-viskosen Öls verbleiben, welches direkt weiter umgesetzt wird. Destillation einer Probe im Kugelrohrofen ergibt einen Siedepunkt von 260 °C/0,1 mbar.

In analoger Weise erhält man weitere Verbindungen der Formel (I)

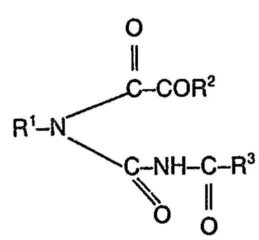

(I)

| Bsp.-Nr. | $R^1$ | $R^2$ | $R^3$ | Physikal. Daten [Schmelzpkt: °C; Brechungsindex: $n_D^{20}$] |
|---|---|---|---|---|
| 2 | 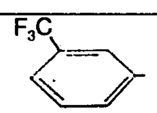 | 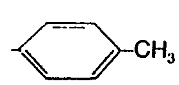 | 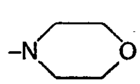 | 102 |

(Fortsetzung)

| Bsp.-Nr. | $R^1$ | $R^2$ | $R^3$ | Physikal. Daten [Schmelzpkt: °C; Brechungsindex: $n_D^{20}$] |
|---|---|---|---|---|
| 3 | 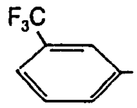 | 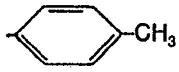 | $-OCH_3$ | 72 |
| 4 | 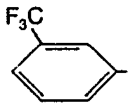 | 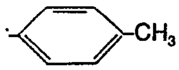 | $-OC_6H_{13}-n$ | 69 |
| 5 | 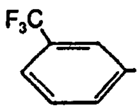 | 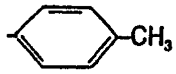 | $-OC_{10}H_{21}-n$ | 1.4930 |
| 6 | 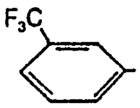 | 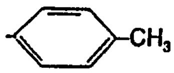 | $-OCH_2CH_2OCH_3$ | 1.5280 |
| 7 | 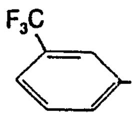 | 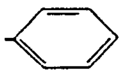 | 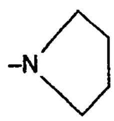 | 59 |
| 8 | 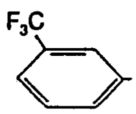 | 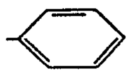 | 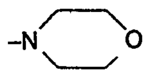 | 121 |
| 9 | 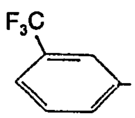 | 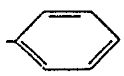 | $-OCH_2CH_2-OCH_3$ | 1.5120 |
| 10 | 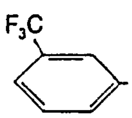 | 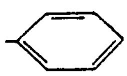 | $-OC_2H_5$ | 1.5100 |
| 11 | 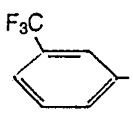 | 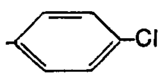 | $-OCH_3$ | 1.5170 |
| 12 | 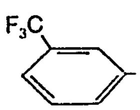 | 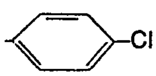 | $-OC_2H_5$ | 66 |
| 13 | 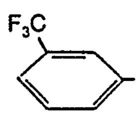 | 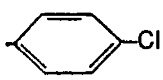 | $-OC_4H_9-n$ | 1.5140 |
| 14 | 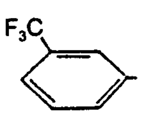 | 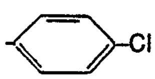 | $-OC_{10}H_{21}-n$ | hochviskos |
| 15 | 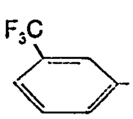 | 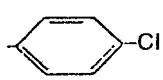 | 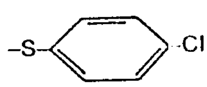 | 68 |
| 16 | 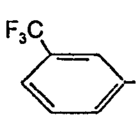 | 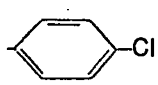 | 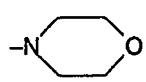 | 155 |

(Fortsetzung)

| Bsp.-Nr. | $R^1$ | $R^2$ | $R^3$ | Physikal. Daten [Schmelzpkt: °C; Brechungsindex: $n_D^{20}$] |
|---|---|---|---|---|
| 17 | 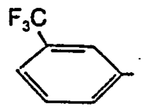 | 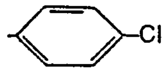 | 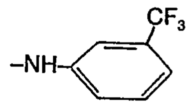 | 1.5280 |
| 18 | 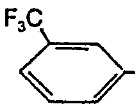 | 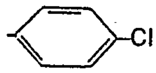 | 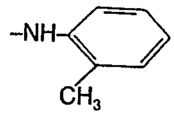 | 180 |
| 19 | 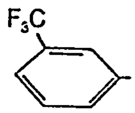 | 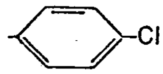 | 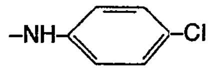 | 160 |
| 20 | 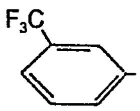 | 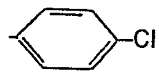 | 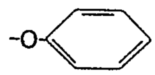 | 150 |
| 21 | 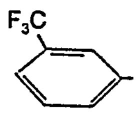 | 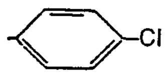 | 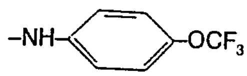 | 1.542 |
| 22 | 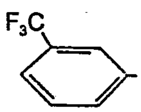 | 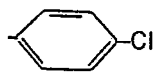 | $-NH-CH_2-C(CH_3)_3$ | 77 |
| 23 | 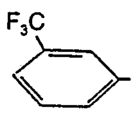 | 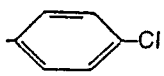 | $-NH-C(CH_3)_3$ | 95 |
| 24 | 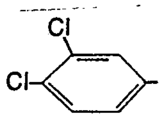 | 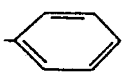 | $-NH-C(CH_3)_3$ | 1.505 |
| 25 | 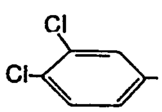 | 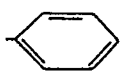 | $-NH-CH_2-C(CH_3)_3$ | 60 |
| 26 | 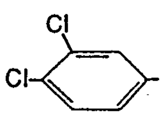 | 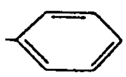 | 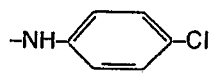 | 89 |
| 27 | 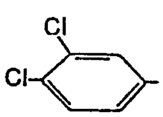 | 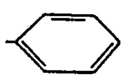 | 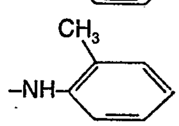 | 165 |
| 28 | 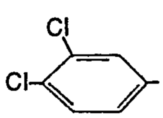 | 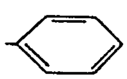 | 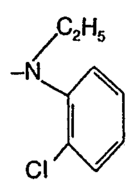 | 87 |
| 29 | 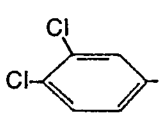 | 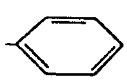 | $-OC_4H_9-n$ | 108 |
| 30 | 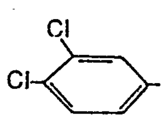 | 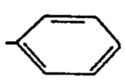 | $-OCH(CH_3)_2$ | 143 |

(Fortsetzung)

| Bsp.-Nr. | $R^1$ | $R^2$ | $R^3$ | Physikal. Daten [Schmelzpkt: °C; Brechungsindex: $n_D^{20}$] |
|---|---|---|---|---|
| 31 | 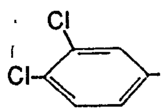 | 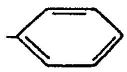 | $-OC_2H_5$ | 100 |
| 32 | 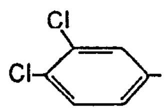 | 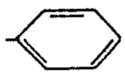 | $-OCH_3$ | 112 |
| 33 | 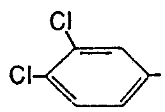 | 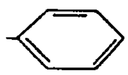 | 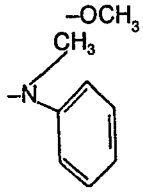 | 115 |
| 34 | $(CH_3)_3C-CH_2-$ | 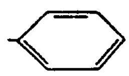 | 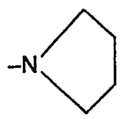 | 1.5130 |
| 35 | $(CH_3)_3C-CH_2-$ | 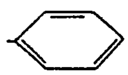 | 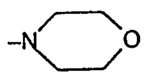 | 92–95 |
| 36 | $(CH_3)_3C-CH_2-$ | 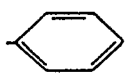 | $N(C_2H_5)_2$ | 1.5000 |
| 37 | $(CH_3)_3C-CH_2-$ | 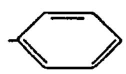 | $-OCH_3$ | 108–111 |
| 38 | $(CH_3)_3C-CH_2-$ | 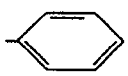 | $-OC_6H_{13}-n$ | 1.4970 |
| 39 | $(CH_3)_3C-CH_2-$ | 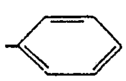 | $OCH_2CH_2-OCH_3$ | 1.5060 |
| 40 | $(CH_3)_3C-CH_2-$ | 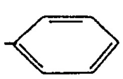 | 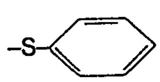 | 100 |
| 41 | $(CH_3)_3C-CH_2-$ | 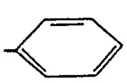 | 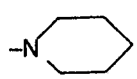 | 1.5200 |
| 42 | $(CH_3)_3C-CH_2-$ | 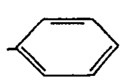 | $-NH-CH_2$<br>$(CH_3)_3C$ | 1.5050 |
| 43 | $(CH_3)_3C-CH_2-$ | 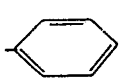 | 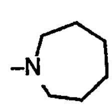 (7-Ring) | hochvisk. Öl |
| 44 | $(CH_3)_3C-CH_2-$ | 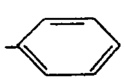 | 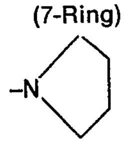 | 1.5510 |

(Fortsetzung)

| Bsp.-Nr. | $R^1$ | $R^2$ | $R^3$ | Physikal. Daten [Schmelzpkt: °C; Brechungsindex: $n_D^{20}$] |
|---|---|---|---|---|
| 45 | $(CH_3)_3C-CH_2-$ | 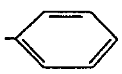 | 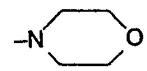 | 1.5390 |
| 46 | $Cl-CH_2-CH_2-$ | 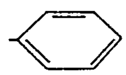 | $-N(C_2H_5)_2$ | 1.5170 |
| 47 | $Cl-CH_2-CH_2-$ | 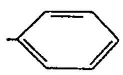 | $-OCH_3$ | 1.5260 |
| 48 | $Cl-CH_2-CH_2-$ | 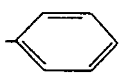 | $-OC_6H_{13}-n$ | 1.5030 |
| 49 | $Cl-CH_2-CH_2-$ | 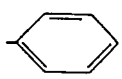 | $-OCH_2-CH_2-OCH_3$ | 1.5190 |
| 50 | $Cl-CH_2-CH_2-$ | 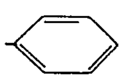 | 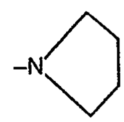 | 1.5360 |
| 51 | $Cl-CH_2-CH_2-$ | 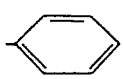 | $-NH-CH_2-C(CH_3)_3$ | 1.5090 |

In den nachfolgenden Beispielen wird die nachstehend aufgeführte Verbindung als Vergleichssubstanz eingesetzt:

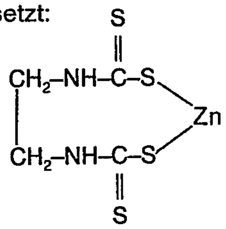

Zink-ethylen-bis-dithiocarbamat

Beispiel A

Pyricularia-Test (Reis)/protektiv

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wässrigen Sporensuspension von Pyricularia oryzae inokuliert. Anschliessend werden die Pflanzen in einem Gewächshaus bei 100% rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäss den Herstellungsbeispielen 14 und 27.

Beispiel B

Pyricularia-Test (Reis)/systemisch

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wässrigen Sporensuspension von Pyricularia oryzae inokuliert. Danach verbleiben die Pflanzen in einem Gewächshaus bei einer Temperatur von 25 °C und einer rel. Luftfeuchtigkeit von 100% bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemäss Herstellungsbeispielen 2, 3, 4 und 8.

**Patentansprüche**

1. Allophanat-Derivate der Formel (I)

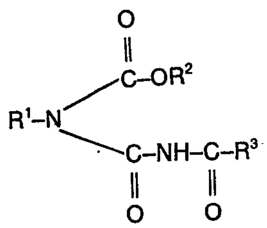

(I)

in welcher

$R^1$ und $R^2$ gleich oder verschieden sind und für gegebenenfalls durch $C_{1-4}$-Alkoxy, $C_{1-4}$-Alkylthio und/oder Halogen substituiertes $C_1$–$C_{10}$-Alkyl oder $C_2$–$C_{10}$-Alkenyl oder $C_2$–$C_{10}$-Alkinyl stehen, ferner für gegebenenfalls durch $C_1$–$C_6$-Alkyl substituiertes $C_5$–$C_{10}$-Cycloalkyl, für gegebenenfalls im Arylteil durch Halogen, Nitro, $C_1$–$C_6$-Alkyl, Cyano und/oder Trifluormethyl substituiertes Aralkyl mit $C_6$–$C_{10}$ im Arylteil und $C_1$–$C_4$ im Alkylteil stehen, für gegebenenfalls durch Halogen, Nitro, Cyano, $C_1$–$C_6$-Alkyl, $C_1$–$C_6$-Alkoxy, und/oder Trifluormethyl substituiertes $C_6$–$C_{10}$-Aryl stehen,

$R^3$ für die Reste $-XR^4$ oder $-NR^5R^6$ steht, wobei

$R^4$ für $C_{1-10}$-Alkyl, für $C_{1-4}$-Alkoxy-$C_{1-4}$-alkyl oder für gegebenenfalls ein- bis fünffach, gleich oder verschieden durch Halogen, $C_{1-4}$-Alkyl oder Halogenalkyl mit 1 oder 2 Kohlenstoffatomen und 1 bis 5 gleichen oder verschiedenen Halogenatomen substituiertes Aryl mit 6 bis 10 Kohlenstoffatomen steht,

X für Sauerstoff oder Schwefel steht und

$R^5$ und $R^6$ gleich oder verschieden sind und für Wasserstoff, für $C_{1-10}$-Alkyl oder für gegebenenfalls ein- bis fünffach, gleich oder verschieden durch Halogen, $C_{1-4}$-Alkyl, Halogenalkyl oder Halogenalkoxy mit je 1 oder 2 Kohlenstoffatomen und 1 bis 5 gleichen oder verschiedenen Halogenatomen substituiertes Aryl mit 6 bis 10 Kohlenstoffatomen stehen oder

$R^5$ und $R^6$ gemeinsam mit dem Stickstoffatom, an dem sie stehen, für einen 5- bis 7-gliedrigen gesättigten, gegebenenfalls durch $C_{1-4}$-Alkyl substituierten Heterocyclus stehen, der durch ein weiteres Heteroatom unterbrochen sein kann,

ausgenommen die Verbindung N-Carbethoxy-N-methyl-N′-phenoxycarbonylharnstoff.

2. Allophanat-Derivate gemäss Anspruch 1, wobei in der Formel (I)

$R^1$ und $R^2$ gleich oder verschieden sind und für gegebenenfalls durch Methoxy, Ethoxy, n- und i-Propoxy, Methylthio, Ethylthio, n- und i-Propylthio, Fluor und/oder Chlor substituiertes $C_1$–$C_6$-Alkyl oder $C_3$–$C_5$-Alkenyl oder $C_3$–$C_5$-Alkinyl steht, für gegebenenfalls durch Methyl, Ethyl, n- und i-Propyl, n-, s-, i- und t-Butyl substituiertes $C_5$- oder $C_6$-Cycloalkyl, für gegebenenfalls im Phenylteil durch Fluor, Chlor, Nitro, Cyano, Methyl, Ethyl, n- und i-Propyl, n-, s-, i- und t-Butyl und Trifluormethyl substituiertes Benzyl oder Phenylethyl stehen, gegebenenfalls durch Fluor, Chlor, Nitro, Methyl, Ethyl, n- und i-Propyl, n-, i-, s- und t-Butyl, Methoxy, Ethoxy, n- und i-Propoxy, und/oder Trifluormethyl substituiertes Phenyl oder Naphthyl stehen,

$R^3$ für die Reste $-XR^4$ oder $-NR^5R^6$ steht, wobei

$R^4$ für $C_{1-10}$-Alkyl, für $C_{1-3}$-Alkoxy-$C_{1-4}$-alkyl oder gegebenenfalls ein- bis dreifach, gleich oder verschieden durch Chlor, Methyl, Ethyl oder Trihalogenmethyl substituiertes Phenyl steht,

X für Sauerstoff oder Schwefel steht,

$R^5$ und $R^6$ gleich oder verschieden sind und für Wasserstoff, $C_{1-6}$-Alkyl oder für gegebenenfalls ein- bis dreifach, gleich oder verschieden durch Chlor, Methyl, Ethyl, Trihalogenmethyl, Trihalogenmethoxy substituiertes Phenyl stehen oder

$R^5$ und $R^6$ gemeinsam mit dem Stickstoffatom, an dem sie stehen, einen gesättigten, gegebenenfalls ein- oder zweifach durch Methyl substituierten 5- bis 7-gliedrigen Heterocyclus bilden, der durch ein Sauerstoffatom unterbrochen sein kann, ausgenommen die Verbindung N-Carbethoxy-N-methyl-N′-phenoxycarbonylharnstoff.

3. Allophanat-Derivate gemäss Anspruch 1, wobei in der Formel (I)

$R^1$ für $C_{1-6}$-Alkyl, Cyclopentyl, Cyclohexyl, gegebenenfalls ein- oder zweifach durch Chlor, Methyl, tert.-Butyl oder Trifluormethyl substituiertes Phenyl oder Benzyl steht,

$R^2$ für $C_{1-4}$-Alkyl, für gegebenenfalls ein- bis dreifach, gleich oder verschieden durch Methyl, Chlor oder iso-Propoxy substituiertes Phenyl steht,

$R^3$ für die Reste $-XR^4$ oder $-NR^5R^6$ steht, wobei

$R^4$ für $C_{1-10}$-Alkyl, für $C_{1-3}$-Alkoxy-$C_{1-4}$-alkyl oder gegebenenfalls ein- bis dreifach, gleich oder verschieden durch Chlor, Methyl, Ethyl oder Trifluormethyl substituiertes Phenyl steht,

X für Sauerstoff oder Schwefel steht,

$R^5$ und $R^6$ gleich oder verschieden sind und für Wasserstoff, $C_{1-6}$-Alkyl oder für gegebenenfalls ein- bis dreifach, gleich oder verschieden durch Chlor, Methyl, Ethyl, Trifluormethyl oder Trifluormethoxy substituiertes Phenyl stehen oder

$R^5$ und $R^6$ gemeinsam mit dem Stickstoffatom, an dem sie stehen, für einen gegebenenfalls ein- oder zweifach durch Methyl substituierten Morpholinrest, Pyrrolidinrest, Hexamethylenimidrest oder Piperidinrest stehen,

ausgenommen die Verbindung N-Carbethoxy-N-methyl-N′-phenoxycarbonylharnstoff.

4. Verfahren zur Herstellung von Allophanat-Derivaten der Formel (I)

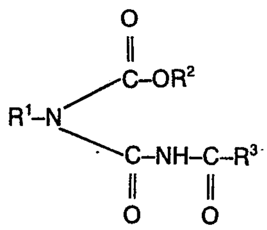

(I)

in welcher

$R^1$ und $R^2$ gleich oder verschieden sind und für gegebenenfalls durch $C_{1-4}$-Alkoxy, $C_{1-4}$-Alkylthio und/oder Halogen substituiertes $C_1$–$C_{10}$-Alkyl oder $C_2$–$C_{10}$-Alkenyl oder $C_2$–$C_{10}$-Alkinyl stehen, ferner für gegebenenfalls durch $C_1$–$C_6$-Alkyl substituiertes $C_5$–$C_{10}$-Cycloalkyl, für gegebenenfalls im Arylteil durch Halogen, Nitro, $C_1$–$C_6$-Alkyl, Cyano und/oder Trifluormethyl substituiertes Aralkyl mit $C_6$–$C_{10}$ im Arylteil und $C_1$–$C_4$ im Alkylteil stehen, für gegebenenfalls durch Halogen, Nitro, Cyano, $C_1$–$C_6$-Alkyl, $C_1$–$C_6$-Alkoxy, und/oder Trifluormethyl substituiertes $C_6$–$C_{10}$-Aryl stehen,

$R^3$ für die Reste $-XR^4$ oder $-NR^5R^6$ steht, wobei

$R^4$ für $C_{1-10}$-Alkyl, für $C_{1-4}$-Alkoxy-$C_{1-4}$-alkyl oder für gegebenenfalls ein- bis fünffach, gleich oder verschieden durch Halogen, $C_{1-4}$-Alkyl oder Halogenalkyl mit 1 oder 2 Kohlenstoffatomen und 1 bis 5 gleichen oder verschiedenen Halogenatomen substituiertes Aryl mit 6 bis 10 Kohlenstoffatomen steht,

X für Sauerstoff oder Schwefel steht und

$R^5$ und $R^6$ gleich oder verschieden sind und für Wasserstoff, für $C_{1-10}$-Alkyl oder für gegebenenfalls ein- bis fünffach, gleich oder verschieden durch Halogen, $C_{1-4}$-Alkyl, Halogenalkyl oder Halogenalkoxy mit je 1 oder 2 Kohlenstoffatomen und 1 bis 5 gleichen oder verschiedenen Halogenatomen substituiertes Aryl mit 6 bis 10 Kohlenstoffatomen stehen oder

$R^5$ und $R^6$ gemeinsam mit dem Stickstoffatom, an dem sie stehen, für einen 5- bis 7-gliedrigen gesättigten, gegebenenfalls durch $C_{1-4}$-Alkyl substituierten Heterocyclus stehen, der durch ein weiteres Heteroatom unterbrochen sein kann,

ausgenommen die Verbindung N-Carbethoxy-N-methyl-N'-phenoxycarbonylharnstoff, dadurch gekennzeichnet, dass man

a) N-Isocyanatocarbonyl-carbamate der Formel (II)

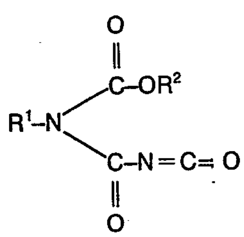

(II)

in welcher

$R^1$ und $R^2$ die oben angegebene Bedeutung haben, mit Verbindungen der Formel (III)

$$MR^3 \quad (III)$$

in welcher

$R^3$ die oben angegebene Bedeutung hat und

M für Wasserstoff oder ein Metallkationäquivalent steht,

gegebenenfalls in Gegenwart eines basischen Hilfsstoffes und gegebenenfalls in Gegenwart eines Verdünnungs- oder Lösungsmittels umsetzt, oder dass man

b) N-Halogencarbonyl-urethane der Formel (IV)

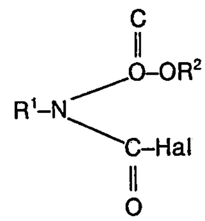

(IV)

in welcher

$R^1$ und $R^2$ die oben angegebene Bedeutung haben und

Hal für Halogen steht, mit Verbindungen der Formel (V)

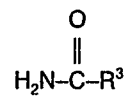

(V)

in welcher

$R^3$ die oben angegebene Bedeutung hat,

gegebenenfalls in Gegenwart eines Lösungs- oder Verdünnungsmittels und gegebenenfalls in Gegenwart einer Base umsetzt.

5. Schädlingsbekämpfungsmittel, gekennzeichnet durch einen Gehalt an mindestens einem Allophanat-Derivat der Formel (I) gemäss den Ansprüchen 1 und 4.

6. Verwendung von Allophanat-Derivaten der Formel (I) gemäss den Ansprüchen 1 und 4 zur Bekämpfung von Schädlingen.

7. Verfahren zur Bekämpfung von Schädlingen, dadurch gekennzeichnet, dass man Allophanat-Derivate der Formel (I) gemäss den Ansprüchen 1 und 4 auf Schädlinge und/oder ihren Lebensraum einwirken lässt.

8. Verfahren zur Herstellung von Schädlingsbekämpfungsmitteln, dadurch gekennzeichnet, dass man Allophanat-Derivate der Formel (I) gemäss den Ansprüchen 1 und 4 mit Streckmitteln und/oder oberflächenaktiven Mitteln vermischt.

**Claims**

1. Allophanate derivatives of the formula (I)

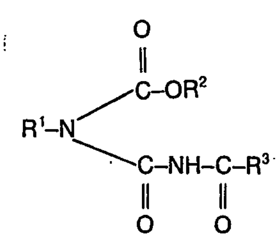

(I)

in which

$R^1$ and $R^2$ are identical or different and represent $C_1$–$C_{10}$-alkyl or $C_2$–$C_{10}$-alkenyl or $C_2$–$C_{10}$-alkinyl which is optionally substituted by $C_{1-4}$-alkoxy, $C_{1-4}$-alkylthio and/or halogen, furthermore $C_5$–$C_{10}$-cycloalkyl which is optionally substituted by $C_1$–$C_6$-alkyl, aralkyl with $C_6$–$C_{10}$ in the aryl part and $C_1$–$C_4$ in the alkyl part which is optionally substituted in the aryl part by halogen, nitro, $C_1$–$C_6$-alkyl, cyano and/or trifluoromethyl, $C_6$–$C_{10}$-aryl which is optionally substituted by halogen,

nitro, cyano, $C_1$–$C_6$-alkyl, $C_1$–$C_6$-alkoxy and/or trifluoromethyl

$R^3$ represents the radical $-XR^4$ or $-NR^5R^6$, wherein

$R^4$ represents $C_{1-10}$-alkyl, $C_{1-4}$-alkoxy-$C_{1-4}$-alkyl, or aryl with 6 to 10 carbon atoms which is optionally mono- to pentasubstituted by identical or different substituents from the group comprising halogen, $C_{1-4}$-alkyl and halogenalkyl with 1 to 2 carbon atoms and 1 to 5 identical or different halogen atoms,

X represents oxygen or sulphur and

$R^5$ and $R^6$ are identical or different and represent hydrogen, $C_{1-10}$-alkyl, or aryl with 6 to 10 carbon atoms which is optionally mono to pentasubstituted, by identical or different substituents from the group comprising halogen, $C_{1-4}$-alkyl, halogenalkyl and halogenoalkoxy with in each case 1 or 2 carbon atoms and 1 to 5 identical or different halogenatoms, or $R^5$ and $R^6$, together with the nitrogen atom on which they stand, represent a 5-membered to 7-membered saturated heterocyclic radical which is optionally substituted by $C_{1-4}$-alkyl and can be interrupted by a further hetero atom,

except the compound N-carbethoxy-N-methyl-N'-phenoxycarbonyl urea.

2. Allophanate derivatives according to Claim 1, wherein in formula (I)

$R^1$ and $R^2$ are identical or different and represent $C_1$–$C_6$-alkyl or $C_3$–$C_5$-alkenyl or $C_3$–$C_5$-alkinyl, optionally substituted by methoxy, ethoxy, n- and i-propoxy, methylthio, ethylthio, n- and i-propylthio, fluorine and/or chlorine, $C_5$- or $C_6$-cycloalkyl, optionally substituted by methyl, ethyl, n- and i-propyl, n-, s-, i- and t-butyl, benzyl or phenylethyl, optionally substituted in the phenyl part by fluorine, chlorine, nitro, cyano, methyl, ethyl, n- and i-propyl, n-, s-, i- and t-butyl and trifluoromethyl, phenyl or naphthyl, optionally substituted by fluorine, chlorine, nitro, methyl, ethyl, n- and i-propyl, n-, i-, s- and t-butyl, methoxy, ethoxy, n- and i-propoxy and/or trifluoromethyl,

$R^3$ represents the radical $-XR^4$ or $-NR^5R^6$, wherein

$R^4$ represents $C_{1-10}$-alkyl, $C_{1-3}$-alkoxy-$C_{1-4}$-alkyl, or phenyl which is optionally mono-, di- or trisubstituted by identical or different substituents from the group comprising chlorine, methyl, ethyl and trihalogenomethyl,

X represents oxygen or sulphur and

$R^5$ and $R^6$ are identical or different and represent hydrogen, $C_1$–$C_6$-alkyl or phenyl which is optionally mono-, di- or trisubstituted by identical or different substituents from the group comprising chlorine, methyl, ethyl, trihalogenomethyl and trihalogenomethoxy or

$R^5$ and $R^6$, together with the nitrogen atom on which they stand, form a 5-membered to 7-membered saturated heterocyclic radical, which is optionally mono- or disubstituted by methyl and can be interrupted by an oxygen atom,

except the compound N-carbethoxy-N-methyl-N'-phenoxycarbonyl urea.

3. Allophanate derivatives according to Claim 1, wherein, in formula (I),

$R^1$ represents $C_1$–$C_6$-alkyl, cyclopentyl, cyclohexyl, or phenyl or benzyl which is optionally mono- or disubstituted by chlorine, methyl, tert.-butyl or trifluoromethyl,

$R^2$ represents $C_{1-4}$-alkyl or phenyl which is optionally mono-, di- or trisubstituted by identical or different substituents from the group comprising methyl, chlorine and iso-propoxy,

$R^3$ represents the radical $-XR^4$ or $-NR^5R^6$, wherein

$R^4$ represents $C_{1-10}$-alkyl, $C_{1-3}$-alkoxy-$C_{1-4}$-alkyl, or phenyl which is optionally mono-, di- or trisubstituted by identical or different substituents from the group comprising chlorine, methyl, ethyl and trifluoromethyl,

X represents oxygen or sulphur

$R^5$ and $R^6$ are identical or different and represent hydrogen, $C_{1-6}$-alkyl, or phenyl which is optionally mono-, di- or trisubstituted by identical or different substituents from the group comprising chlorine, methyl, ethyl, trifluoromethyl and trifluoromethoxy, or

$R^5$ and $R^6$, together with the nitrogen atom on which they stand, represent a morpholine radical, pyrrolidine radical, hexamethyleneimide radical or piperidine radical which is optionally mono- or disubstituted by methyl,

except the compound N-carbethoxy-N-methyl-N'-phenoxy-carbonyl urea.

4. Process for the preparation of allophanate derivatives of the formula (I)

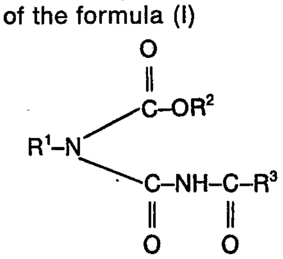

(I)

wherein, in formula (I),

$R^1$ and $R^2$ are identical or different and represent $C_1$–$C_{10}$-alkyl or $C_2$–$C_{10}$-alkenyl or $C_2$–$C_{10}$-alkinyl which is optionally substituted by $C_{1-4}$-alkoxy, $C_{1-4}$-alkylthio and/or halogen, furthermore $C_5$–$C_{10}$-cycloalkyl which is optionally substituted by $C_1$–$C_6$-alkyl, aralkyl with $C_6$–$C_{10}$ in the aryl part and $C_1$–$C_4$ in the alkyl part which is optionally substituted in the aryl part by halogen, nitro, $C_1$–$C_6$-alkyl, cyano and/or trifluoromethyl, $C_6$–$C_{10}$-aryl which is optionally substituted by halogen, nitro, cyano, $C_1$–$C_6$-alkyl, $C_1$–$C_6$-alkoxy and/or trifluoromethyl

$R^3$ represents the radical $-XR^4$ or $-NR^5R^6$, wherein

$R^4$ represents $C_{1-10}$-alkyl, $C_{1-4}$-alkoxy-$C_{1-4}$-alkyl, or aryl with 6 to 10 carbon atoms which is optionally mono- to pentasubstituted by identical or different substituents from the group comprising halogen, $C_{1-4}$-alkyl or halogenoalkyl with 1 or 2 carbon atoms and 1 to 5 identical or different halogen atoms,

X represents oxygen or sulphur and

$R^5$ and $R^6$ are identical or different and repre-

sent hydrogen, $C_{1-10}$-alkyl, or aryl with 6 to 10 carbon atoms which is optionally mono to pentasubstituted by identical or different substituents from the group comprising halogen, $C_{1-4}$-alkyl, halogenoalkyl and halogenoalkoxy with in each case 1 or 2 carbon atoms and 1 to 5 identical or different halogenatoms, or

$R^5$ and $R^6$, together with the nitrogen atom on which they stand, represent a saturated 5-membered to 7-membered heterocyclic radical which is optionally substituted by $C_{1-4}$-alkyl and can be interrupted by a further heteroatom,

except the compound N-carbethoxy-N-methyl-N'-phenoxycarbonyl urea, characterized in that

a) N-isocyanatocarbonyl-carbamates of the formula (II)

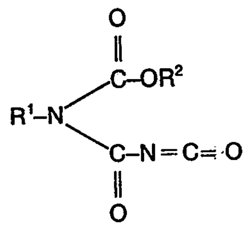 (II)

in which

$R^1$ and $R^2$ have the abovementioned meaning, are reacted with compounds of the formula (III)

$$MR^3 \qquad (III)$$

in which

$R^3$ has the abovementioned meaning and

M represents hydrogen or one metal cation equivalent,

if appropriate in the presence of a basic auxiliary and if appropriate in the presence of a diluent or solvent, or in that

b) N-halogenocarbonyl-urethanes of the formula (IV)

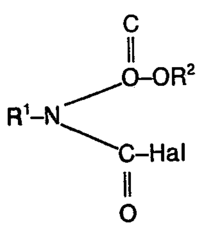 (IV)

in which

$R^1$ and $R^2$ have the abovementioned meaning and

Hal represents halogen, are reacted with compounds of the formula (V)

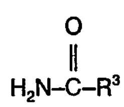 (V)

in which

$R^3$ has the abovementioned meaning,

if appropriate in the presence of a solvent or diluent and if appropriate in the presence of a base.

5. Agents for combating pests, characterized in that they contain at least one allophanate derivative of the formula (I) according to Claims 1 and 4.

6. Use of allophanate derivatives of the formula (I) according to Claims 1 and 4 for combating pests.

7. Process for combating pests, characterized in that allophanate derivatives of the formula (I) according to Claims 1 and 4 are allowed to act on pests and/or their environment.

8. Process for the preparation of agents for combating pests, characterized in that allophanate derivatives of the formula (I) according to Claims 1 and 4 are mixed with extenders and/or surface-active agents.

**Revendications**

1. Dérivés d'allophanates de formule (I)

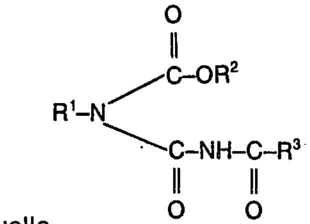 (I)

dans laquelle

$R^1$ et $R^2$, ayant des significations identiques ou différentes, représentent chacun un groupe alkyle en $C_1$–$C_{10}$ ou alcényle en $C_2$–$C_{10}$ ou alcynyle en $C_2$–$C_{10}$ éventuellement substitué par des groupes alcoxy en $C_1$–$C_4$, alkylthio en $C_1$–$C_4$ et/ou des halogènes, ou bien un groupe cycloalkyle en $C_5$–$C_{10}$ éventuellement substitué par des groupes alkyles en $C_1$–$C_6$, un groupe aralkyle, contenant 6 à 10 atomes de carbone dans la partie aryle et 1 à 4 atomes de carbone dans la partie alkyle, éventuellement substitué dans la partie aryle par des halogènes, des groupes nitro, alkyle en $C_1$–$C_6$, cyano et/ou trifluorométhyle, ou un groupe aryle en $C_6$–$C_{10}$ éventuellement substitué par des halogènes, des groupes nitro, cyano, alkyle en $C_1$–$C_6$, alcoxy en $C_1$–$C_6$ et/ou trifluorométhyle,

$R^3$ représente un groupe $-XR^4$ ou $-NR^5R^6$ dans lequel

$R^4$ représente un groupe alkyle en $C_1$–$C_{10}$, un groupe (alcoxy en $C_1$–$C_4$)-alkyle en $C_1$–$C_4$ ou un groupe aryle en $C_6$–$C_{10}$ portant éventuellement 1 à 5 substituants identiques ou différents choisis parmi les halogènes, les groupes alkyles en $C_1$–$C_4$ ou halogénoalkyles contenant 1 ou 2 atomes de carbone et 1 à 5 atomes d'halogènes identiques ou différents,

X représente l'oxygène ou le soufre et

$R^5$ et $R^6$, ayant des significations identiques ou différentes, représentent chacun l'hydrogène, un groupe alkyle en $C_1$–$C_{10}$ ou un groupe aryle en $C_6$–$C_{10}$ portant éventuellement 1 à 5 substituants identiques ou différents choisis parmi les halogènes, les groupes alkyles en $C_1$–$C_4$, halogénoalkyles ou halogénoalcoxy contenant chacun 1 ou 2 atomes de carbone et 1 à 5 atomes d'halogènes identiques ou différents, ou bien

$R^5$ et $R^6$ forment ensemble et avec l'atome d'azote auquel ils sont reliés un hétérocycle saturé de 5 à 7 chaînons éventuellement substitué par des groupes alkyles en $C_1$–$C_4$ et qui peut être interrompu par un autre hétéroatome,

à l'exception du composé: N-carbéthoxy-N-méthyl-N'-phénoxycarbonylurée.

2. Dérivés d'allophanates selon la revendication 1, répondant à la formule (I) dans laquelle

$R^1$ et $R^2$, ayant des significations identiques ou différentes, représentent chacun un groupe alkyle en $C_1$–$C_6$ ou alcényle en $C_3$–$C_5$ ou alcynyle en $C_3$–$C_5$ éventuellement substitué par des groupes méthoxy, éthoxy, n- et isopropoxy, méthylthio, éthylthio, n- et isopropylthio, le fluor et/ou le chlore, un groupe cycloalkyle en $C_5$ ou $C_6$ éventuellement substitué par des groupes méthyle, éthyle, n- et isopropyle, n-, sec-, iso-, et tert-butyle, un groupe benzyle ou phényléthyle éventuellement substitué dans la partie phényle par le fluor, le chlore, des groupes nitro, cyano, méthyle, éthyle, n- et isopropyle, n-, sec-, iso- et tert-butyle et trifluorométhyle, un groupe phényle ou naphtyle éventuellement substitué par le fluor, le chlore, des groupes nitro, méthyle, éthyle, n- et isopropyle, n-, iso-, sec-, et tert-butyle, méthoxy, éthoxy, n- et isopropoxy, et/ou trifluorométhyle,

$R^3$ représente un groupe $-XR^4$ ou $-NR^5R^6$ dans lequel

$R^4$ représente un groupe alkyle en $C_1$–$C_{10}$, (alcoxy en $C_1$–$C_3$)-alkyle en $C_1$–$C_4$ ou un groupe phényle portant éventuellement 1 ou 3 substituants identiques ou différents choisis parmi le chlore, les groupes méthyle, éthyle ou trihalogénométhyle,

X représente l'oxygène ou le soufre,

$R^5$ et $R^6$, ayant des significations identiques ou différentes, représentent chacun l'hydrogène, un groupe alkyle en $C_1$–$C_6$ ou un groupe phényle portant éventuellement 1 à 3 substituants identiques ou différents choisis parmi le chlore, les groupes méthyle, éthyle, trihalogénométhyle, trihalogénométhoxy, ou bien

$R^5$ et $R^6$ forment ensemble et avec l'atome d'azote auquel ils sont reliés un hétérocycle saturé de 5 à 7 chaînons éventuellement mono- ou disubstitué par des groupes méthyle, et qui peut être interrompu par un atome d'oxygène,

à l'exception du composé: N-carbéthoxy-N-méthyl-N'-phénoxycarbonylurée.

3. Dérivés d'allophanates selon la revendication 1 répondant à la formule (I) dans laquelle

$R^1$ représente un groupe alkyle en $C_1$–$C_6$, cyclopentyle, cyclohexyle, phényle ou benzyle éventuellement mono- ou disubstitué par le chlore, des groupes méthyle, tert-butyle ou trifluorométhyle,

$R^2$ représente un groupe alkyle en $C_1$–$C_4$ ou un groupe phényle portant éventuellement 1 à 3 substituants identiques ou différents choisis parmi les groupes méthyle, le chlore ou les groupes isopropoxy,

$R^3$ représente un groupe $-XR^4$ ou $-NR^5R^6$ dans lequel

$R^4$ représente un groupe alkyle en $C_1$–$C_{10}$, un groupe (alcoxy en $C_1$–$C_3$)-alkyle en $C_1$–$C_4$, ou un groupe phényle portant éventuellement 1 à 3 substituants identiques ou différents choisis parmi le chlore, les groupes méthyle, éthyle ou trifluorométhyle,

X représente l'oxygène ou le soufre,

$R^5$ et $R^6$, ayant des significations identiques ou différentes représentent chacun l'hydrogène, un groupe alkyle en $C_1$–$C_6$ ou un groupe phényle portant éventuellement 1 à 3 substituants identiques ou différents choisis parmi le chlore, les groupes méthyle, éthyle, trifluorométhyle ou trifluorométhoxy, ou bien

$R^5$ et $R^6$ forment ensemble et avec l'atome d'azote auquel ils sont reliés un radical de morpholine, un radical de pyrrolidine, un radical d'hexaméthylène-imide ou un radical de pipéridine, éventuellement mono- ou disubstitué par des groupes méthyle,

à l'exception du composé: N-carbéthoxy-N-méthyl-N'-phénoxycarbonylurée.

4. Procédé de préparation des dérivés d'allophanates de formule (I)

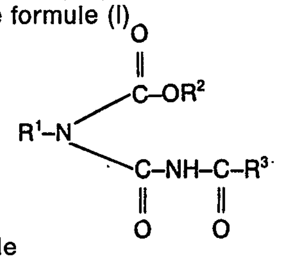

(I)

dans laquelle

$R^1$ et $R^2$, ayant des significations identiques ou différentes, représentent chacun un groupe alkyle en $C_1$–$C_{10}$ ou alcényle en $C_2$–$C_{10}$ ou alcynyle en $C_2$–$C_{10}$, éventuellement substitué par des groupes alcoxy en $C_1$–$C_4$, alkylthio en $C_1$–$C_4$ et/ou des halogènes, ou bien un groupe cycloalkyle en $C_5$–$C_{10}$ éventuellement substitué par des groupes alkyle en $C_1$–$C_6$, un groupe aralkyle contenant 6 à 10 atomes de carbone dans la partie aryle et 1 à 4 atomes de carbone dans la partie alkyle, éventuellement substitué dans la partie aryle par des halogènes, des groupes nitro, alkyle en $C_1$–$C_6$, cyano et/ou trifluorométhyle, ou un groupe aryle en $C_6$–$C_{10}$ éventuellement substitué par des halogènes, des groupes nitro, cyano, alkyle en $C_1$–$C_6$, alcoxy en $C_1$–$C_6$ et/ou trifluorométhyle,

$R^3$ représente un groupe $-XR^4$ ou $-NR^5R^6$ dans lequel

$R^4$ représente un groupe alkyle en $C_1$–$C_{10}$, un groupe (alcoxy en $C_1$–$C_4$)-alkyle en $C_1$–$C_4$ ou un groupe aryle en $C_6$–$C_{10}$ portant éventuellement 1 à 5 substituants identiques ou différents choisis parmi les halogènes, les groupes alkyles en $C_1$–$C_4$ ou halogénoalkyles contenant 1 ou 2 atomes de carbone et 1 à 5 atomes d'halogènes identiques ou différents,

X représente l'oxygène ou le soufre et,

$R^5$ et $R^6$, ayant des significations identiques ou différentes, représentent chacun l'hydrogène, un groupe alkyle en $C_1$–$C_{10}$ ou un groupe aryle en $C_6$–$C_{10}$ portant éventuellement 1 à 5 substituants identiques ou différents choisis parmi les halogènes, les groupes alkyles en $C_1$–$C_4$, halogénoalkyles ou halogénoalcoxy, contenant chacun 1 ou 2 atomes de carbone et 1 à 5 atomes d'halogènes identiques ou différents, ou bien

$R^5$ et $R^6$ forment ensemble et avec l'atome d'azote auquel ils sont reliés un hétérocycle saturé de 5 à 7 chaînons éventuellement substitué par des groupes alkyles en $C_1$–$C_4$ et qui peut être interrompu par un autre hétéroatome,

à l'exception du composé: N-carbéthoxy-N-méthyl-N'-phénoxycarbonylurée, caractérisé en ce que

a) on fait réagir des N-isocyanatocarbonyl-carbamates de formule (II)

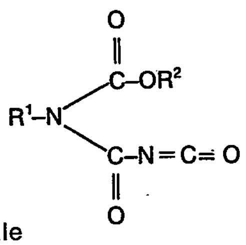

(II)

dans laquelle

$R^1$ et $R^2$ ont les significations indiquées ci-dessus, avec des composés de formule (III),

$MR^3$ (III)

dans laquelle

$R^3$ a les significations indiquées ci-dessus et

M représente l'hydrogène ou un équivalent d'un cation métallique,

éventuellement en présence d'un produit auxiliaire basique et éventuellement en présence d'un diluant ou solvant, ou bien

b) on fait réagir des N-halogénocarbonyl-uréthannes de formule (IV)

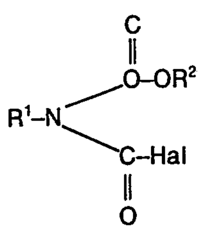

(IV)

dans laquelle

$R^1$ et $R^2$ ont les significations indiquées ci-dessus

et

Hal représente un halogène, avec des composés de formule (V)

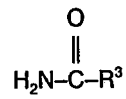

(V)

dans laquelle

$R^3$ a les significations indiquées ci-dessus, éventuellement en présence d'un solvant ou diluant et éventuellement en présence d'une base.

5. Produit pesticide caractérisé en ce qu'il contient au moins un dérivé d'allophanate de formule (I) selon les revendications 1 et 4.

6. Utilisation des dérivés d'allophanates de formule (I) selon les revendications 1 et 4 dans la lutte contre les parasites.

7. Procédé pour combattre les parasites, caractérisé en ce que l'on fait agir sur les parasites et/ou leur habitat des dérivés d'allophanates de formule (I) selon les revendications 1 et 4.

8. Procédé de préparation de produits pesticides, caractérisé en ce que l'on mélange des dérivés d'allophanates de formule (I) selon les revendications 1 et 4 avec des diluants et/ou des agents tensioactifs.